# EUROPEAN PATENT SPECIFICATION

(11) **EP 1 535 622 B1**
(45) Date of publication and mention of the grant of the patent: **31.12.2008**
(21) Application number: 05002951.1
(22) Date of filing: 06.04.2000
(51) Int. Cl.: A61K 38/21, A61K 47/48, A61P 35/00

(54) **Melanoma therapy**
Melanoma Therapie
Thérapie du mélanome

(30) Priority: 08.04.1999 US 288366
(43) Date of publication of application: 01.06.2005
(62) Divisional of application: 00107101.8
(73) Proprietor: Schering Corporation, Kenilworth, NJ 07033-0530 (US)
(72) Inventor: Rybak, Mary Ellen, Warren, NJ 07059 (US); Rose, Esther Helen, Westfield, NJ 07090 (US)
(74) Representative: UEXKÜLL & STOLBERG

(56) References cited:
- WO-A-98/48840
- KIRKWOOD JOHN M ET AL: "Interferon alfa-2b adjuvant therapy of high-risk resected cutaneous melanoma: The eastern cooperative oncology group trial EST 1684." JOURNAL OF CLINICAL ONCOLOGY, vol. 14, no. 1, 1996, pages 7-17, XP000953174 ISSN: 0732-183X
- TALPAZ M ET AL: "Phase I study of polyethylene glycol (PEG) interferon alpha-2B (Intron-A) in CML patients." BLOOD, vol. 92, no. 10 SUPPL. 1 PART 1-2, 15 November 1998 (1998-11-15), page 251A, XP000953118 ISSN: 0006-4971
- TALPAZ M ET AL: "Phase I study of pegylated-interferon alpha-2a (PEGASYSTM) in patients with chronic myelogenous leukemia (CML)." BLOOD, vol. 94, no. 10 SUPPL. 1 PART 1, 15 November 1999 (1999-11-15), page 530a, XP000953172 ISSN: 0006-4971
- BUKOWSKI RONAL ET AL: "Phase I Study of Polyethylene Glycol ( PEG ) Interferon Alpha -2B ( PEG INTRON ) in Patients with Solid Tumors (Meeting abstract)." PROC ANNU MEET AM SOC CLIN ONCOL, vol. 18, May 1999 (1999-05), page A1719, XP000953163

## Description

### BACKGROUND OF THE INVENTION

This invention relates to an improved therapy for treating patients having melanoma after definitive surgical removal of the lesions by administering a therapeutically effective dose of pegylated interferon-alfa for a time sufficient to increase progression-free survival time.

Melanoma incidence is increasing at a rate that exceeds all that for other solid tumors. Patients with primary melanoma of greater than 4 mm or metastatic melanoma involving regional lymph nodes possess a 50 to 90% mortality risk following surgical excision of the primary melanomas.

WO 98/48840 describes polyethylene glycol-interferon alpha conjugates that are useful in methods of treating viral infections, but does not exemplify using these conjugates to treat melanoma. Talpaz. M. et al., Blood 92: 251a (1998) disclosed a Phase study of pegylated interferon alpha in CML patients who were in a state of hematologic resistance to interferon alpha or were unable to tolerate interferon alpha, but does not disclose using pegylated interferon alpha to treat melanoma.

Recently, the Eastern Cooperative Oncology Group (ECOG") published results of the use of interferon alfa-2b in patients with stage III cutaneous melanoma as adjuvant therapy following surgery for deep primary (T4) or regionally metastatic (N1) melanoma (Kirkwood, J.M., et al. J. Clin. Oncol. Vol 14. (1996) pages 4-17.) The interferon alfa-2b therapy used by ECOG involved an induction phase of 20 million IU of interferon alfa-2b per square meter of body surface area (m²) administered intravenously ("IV") daily for five days every week for four weeks followed by maintenance interferon alfa therapy of 10 million IU/m² administered subcutaneously ("SC") three times a week ("TIW") for 48 weeks. A significant improvement in median disease-free survival and overall survival were observed versus control (observation) despite dosage reductions or delays for toxicity in 50% of the patients during the IV induction therapy phase and in 48% of the patients in the SC maintenance phase. Hematologic, neurologic and constitutional toxicities occured among these patients requiring dose reduction or withdrawal from the interferon alfa therapy. Subject compliance with the dosage and dosage regimen during both phases is considered to be important to achieve maximum clinical benefit. Accordingly, there is a need for improved therapy for treating patients having melanoma with higher patient compliance.

### Summary of the Invention

The present invention provides a method of treating a patient having melanoma which has been surgically removed, which comprises administering to such a patient a therapeutically effective dose of pegylated interferon alfa for a time period sufficient to increase the progression-free survival time.

The present invention also provides a method of treating a patient having cutaneous melanoma which has been surgically removed, which comprises administering to said patient an effective amount of pegylated interferon-alfa once a week for a time period sufficient to increase progression-free survival time.

The present invention further provides a method of treating a patient having cutaneous melanoma which has been surgically removed which comprises administering to such a patient about 3.0 micrograms/kg to about 9.0 micrograms/kg of pegylated interferon alfa-2b once a week for a time period sufficient to increase progression-free survival time. In preferred embodiments, 6.0 micrograms per kilogram is dosed weekly to a patient for eight weeks, and 3.0 micrograms per kilogram or less weekly is dosed to the patient for a period of five years minus the eight weeks of initial dosage. If less than 3.0 micrograms per kilogram are dosed to the patient, preferably the dose reduction steps are 3.0-2.0-1.0 micrograms per kilogram.

The present invention further provides a method comprising the step of marketing a therapeutically effective dose of interferon alfa for administration to a patient with melanoma within about 60 days of surgery in a protocol extending for a time period of at least about 100 weeks.

### Detailed Description of the Invention

The present invention provides an improved method of treating patients with melanoma especially those in State IIB (lesions> 4mm, but without positive nodes)and Stage III (lesions> 4mm and node-positive) primary cutaneous melanoma, preferably after surgery for their State IIB or Stage III melanoma. The improved method provides a safer and more efficacious and tolerable adjuvant therapy treatment for melanoma by use of weekly injections of pegylated interferon. The melanoma patients treatable in accordance with the improved method of the present invention include those newly diagnosed with this disease who were free of disease 56 days post surgery but at high risk for systemic recurrence of the disease. The term "high risk patients" as used herein means those melanoma patients with lesions of Breslow thickness >4mm as well as those patients with lesions of any Breslow thickness with primary or recurrent nodal involvement. Melanoma patients intolerant or resistant to interferon alfa therapy are also included. Treatment with pegylated interferon alfa in accordance with the present invention will continue for a minimum of about two years (about 100-104 weeks) and up to five years, unless there is clinical evidence of disease progression, unacceptable toxicity or the patient requests that the therapy be discontinued.

When the pegylated interferon-alfa administered is a pegylated interferon alfa-2b, the therapeutically effective amount of pegylated interferon alfa-2b administered is in the range of about 3.0 to about 9.0 micrograms per kilogram of pegylated interferon alfa-2b administered once a week (QW), preferably in the range of about 4.5 to about 6.5 micrograms per kilogram of pegylated interferon alfa-2b QW, more preferably in the range of about 5.5 to about 6.5 micrograms per kilogram of pegylated interferon alfa-2b QW, and most preferably in the range of about 6.0 micrograms per kilogram of pegylated interferon alfa-2b administered QW.

In preferred embodiments, 6.0 micrograms per kilogram is dosed weekly to a patient for eight weeks, and 3.0 micrograms per kilogram or less weekly is dosed to the patient for a period of five years minus the eight weeks of initial dosage. If less than 3.0 micrograms per kilogram are dosed to the patient, preferably the dose reduction steps are 3.0-2.0-1.0 micrograms per kilogram.

When the pegylated interferon-alfa administered is a pegylated interferon alfa-2a, the therapeutically effective amount of pegylated interferon alfa-2a administered is in the range of about 50 micrograms to about 500 micrograms once a week("QW"), preferably about 200 micrograms to about 250 micrograms QW.

The term "pegylated interferon alfa" as used herein means polyethylene glycol modified conjugates of interferon alfa, preferably interferon alfa-2a and -2b. The preferred polyethylene-glycol-interferon alfa -2b conjugate is PEG₁₂₀₀₀-interferon alfa 2b. The phrases "12,000 molecular weight polyethylene glycol conjugated interferon alpha" and "PEG₁₂₀₀₀-IFN alfa" as used herein mean conjugates such as are prepared according to the methods of International Application No. WO 95/13090 and containing urethane linkages between the interferon alfa-2a or -2b amino groups and polyethylene glycol having an average molecular weight of 12000.

The preferred PEG₁₂₀₀₀-interferon alfa-2b is prepared by attaching a PEG polymer to the epsilon amino group of a lysine residue in the IFN alfa-2b molecule. A single PEG₁₂₀₀₀ molecule is conjugated to free amino groups on an IFN alfa-2b molecule via a urethane linkage. This conjugate is characterized by the molecular weight of PEG₁₂₀₀₀ attached. The PEG₁₂₀₀₀-IFN alfa-2b conjugate is formulated as a lyophilized powder for injection. The objective of conjugation of IFN alfa with PEG is to improve the delivery of the protein by significantly prolonging its plasma half-life, and thereby provide protracted activity of IFN alfa.

The term " interferon-alfa " as used herein means the family of highly homologous species-specific proteins that inhibit viral replication and cellular proliferation and modulate immune response. Typical suitable interferon-alfas include, but are not limited to, recombinant interferon alfa-2b such as Intron-A interferon available from Schering Corporation, Kenilworth, N.J., recombinant interferon alfa-2a such as Roferon interferon available from Hoffmann-La Roche, Nutley, N.J., recombinant interferon alpha-2C such as Berofor alpha 2 interferon available from Boehringer Ingelheim Pharmaceutical, Inc., Ridgefield, CT., interferon alpha-n1, a purified blend of natural alfa interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Patent Nos. 4,897,471 and 4,695,623 (especially Examples 7, 8 or 9 thereof) and the specific product available from Amgen, Inc., Newbury Park, CA, or interferon alfa-n3 a mixture of natural alfa interferons made by Interferon Sciences and available from the Purdue Frederick Co., Norwalk, CT., under the Alferon Tradename. The use of interferon alfa-2a or alpha-2b is preferred. Since interferon alpha-2b, among all interferons, has the broadest approval throughout the world for treating chronic hepatitis C infection, it is most preferred. The manufacture of interferon alpha-2b is described in U.S. Patent No. 4,530,901.

Other interferon alfa conjugates can be prepared by coupling an interferon alfa to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate-based polymers and the like can be used. Such interferon alfa-polymer conjugates are.described in U.S. Patent No. 4,766,106, U.S. Patent No. 4,917,888, European Patent Application No. 0 236 987, European Patent Application Nos. 0 510 356 , 0 593 868 and 0 809 996 ( pegylated interferon alfa-2a) and International Publication No. WO 95/13090.

Pharmaceutical composition of pegylated interferon alfa-suitable for parenteral administration may be formulated with a suitable buffer, e.g., Tris-HCl, acetate or phosphate such as dibasic sodium phosphate/monobasic sodium phosphate buffer, and pharmaceutically acceptable excipients (e.g., sucrose), carriers (e.g. human serum albumin), toxicity agents (e.g. NaCl), preservatives (e.g. thimerosol, cresol or benylalcohol), and surfactants(e.g. tween or polysorabates) in sterile water for injection. The pegylated interferon alfa-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582. The reconstituted aqueous solutions may also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alfa powder in a separate compartment.

The term "patients having melanoma" as used herein means any patient having melanoma and includes treatment-naive patients as well as treatment-experienced patients as well as patients in the Stage IIB or Stage III cutaneous melanoma. All patients having melanoma are preferably treated by wide excision of the primary melanoma lesion prior to initiation of the improved therapy of the present invention.

The term "treatment-naive patients" as used herein means patients with melanoma including newly-diagnosed melanoma patients who have never been treated with any chemotherapeutic drugs, e.g. dacarbazine ("DTIC") or immunotherapy, e.g., IL-2 as well as any interferon, including but not limited to interferon alfa, or pegylated interferon alfa. All treatment-naive patients having melanoma are preferably treated by wide excision of the primary melanoma lesion prior to initiation of the improved therapy of the present invention.

The term "treatment-experienced patients" as used herein means those patients who have initiated some form of chemotherapeutic ; drug,e.g., DTIC or immunotherapy including, but not limited to interferon-alfa, IL-2 and GMCSF. All treatment-experienced patients having melanoma are preferably treated by wide excision of the primary melanoma lesion prior to initiation of the improved therapy of the present invention.

The term "primary cutaneous melanoma" as used herein means histologically proven primary cutaneous melanoma as defined by the current (1992) American Joint Committee on Cancer Staging Criteria ("AJCC"): in the AJCC Manual for Strategy of Cancer (4th edition) Philadelphia PA Lippincott Publishers 1992 and includes (a) node negative stage IIB disease with deep primary melanomas of Breslow depth more than 4 mm and (b)node positive stage III disease defined, as follows: (1) deep primary melanomas of Breslow depth more than 4 mm (designated CS1 PS1: T4N0M0); (2) primary melanomas of any tumor stage in the presence of N1 regional lymph node metastasis detected at elective lymph node dissection with clinically inapparent regional lymph node metastasis (designated CS1 PS2: any TpN1M0); (3) clinically apparent N1 regional lymph node involvement synchronous with primary melanoma of T1-4 (designated CS2 PS2: any TcN1M0); and (4) regional lymph node recurrence at any interval after appropriate surgery for primary melanoma of any depth (designated CS2R: TxrN1M0 recurrent). Patients in groups 1 to 3 were required to enter this study within 56 days of first primary melanoma biopsy. Patients with regional nodal relapse in group 4 were required to enter this study within 42 days of lymphadenectomy.

All patients with stage III melanoma should be treated by wide excision of the primary melanoma lesion.

Patients with clinically positive nodes in the groin, axilla or neck should have a full lymphadenectomy to surgically remove these cites.

All surgery should be completed within 56 days prior to randomization into this clinical study.

The term "progression-free survival time" ("PFST") as used herein means the time from initiation of melanoma treatment in accordance with the present invention to the documentation of disease progression or recurrence by histological or cytological evidence

The progression-free survival time expected for melanoma patients treated in accordance with the method of this invention is at least about 4 years from initiation of the melanoma therapy of this invention; preferably the PFST is in the range of about 30 to about 43 months from initiation of the melanoma therapy of this invention.

The increase in the progression-free survival time expected for melanoma patients treated in accordance with the method of this invention is greater than about 1.0 years to about 1.5 years compared to control (observation).

The following criteria of treatment failure constitute the only acceptable evidence of disease recurrence or progression:

### Lung/Liver:

Positive cytology or biopsy in the presence of a single new lesion or the appearance of multiple lesions consistent with metastatic disease.

### Central Nervous System:

A positive brain CT or MRI scan or Cerebrospinal fluid (CSF) cytology.

### Cutaneous, Subcutaneous and Lymph Node Recurrence:

Positive cytology or biopsy.

### Bone and Other Organs:

Positive cytology or biopsy in the presence of a single new lesion or the appearance of multiple lesions consistent with metastatic disease identified by two different radiologic studies: i.e., positive gallium scan and contrast GI series or ultrasound, x-ray or CT of abdomen for abdominal disease.

The term "prohibited medications" as used herein includes the following:
a) Other chemotherapy, hormonal, immunologic, biologic or radiation therapy.
b) Colony stimulating factors including erythropoietin and G-CSF.
c) Other investigational drugs.
d) Chronic systemic corticosteroid therapy.

Melanoma patients treated in accordance with the method of the present invention should not receive any of the above-listed prohibited medications during the treatment period.

Pegylated interferon-alfa formulations are not effective when administered orally, so the preferred method of administering the pegylated interferon-alfa is parenterally, preferably by subcutaneous, IV, or IM, injection. Of course, other types of administration of both medicaments, as they become available are contemplated, such as by nasal spray, transdermally, by suppository, by sustained release dosage form, and by pulmonary inhalation. Any form of administration will work so long as the proper dosages are delivered without destroying the active ingredient.

The following Clinical Study Design may be used to treat melanoma patients in accordance with the method of the present invention. Many modifications of this Clinical Study Design protocol will be obvious to the skilled clinician, and the following Study Design should not be interpreted as limiting the scope of the method of this invention which is defined by the claims listed hereinafter.

### Clinical Study Design

This is a Phase II/III randomized, controlled, multicenter, open-label study designed to assess he safety, efficacy, and impact on quality of life of PEG Intron (pegylated interferon alfa 2b i.e. PEG₁₂₀₀₀-interferon alfa 2b and INTRON® A (interferon alfa 2b), which are each available from Schering Corporation, Kenilworth, NJ, and the population pharmacokinetics of PEG Intron when given as adjuvant therapy in subjects with resected Stage III node-positive cutaneous melanoma. It is anticipated that approximately 450 subjects will be enrolled, with 225 subjects randomized to each treatment group.

Subjects will enter the study within 56 days of definitive surgery for their Stage III melanoma and will be randomized to one of the two treatment groups shown below. Definitive surgery includes wide surgical excision of the primary melanoma and lymphadenectomy of all clinically positive nodes in the groin, axilla and neck. All surgery should be complete at least 56 days prior to randomization.

### Group A: INTRON® A

20 MIU/m²/day IV 5 days/week × 4 weeks, followed by 10 MIU/m² SC TIW × 48 weeks.

### Induction Therapy: 20 MIU/m²/day IV 5 days a week for 4 weeks

All subjects randomized to Treatment Group A, will begin induction therapy with intravenous INTRON® A, 20 million international units/m²/day, 5 days/week for 4 weeks. Acetaminophen (500-1000 mg) may be given in the clinic 30 minutes prior to receiving the first dose of INTRON® A. Subjects should be observed for 2 hours after the first dose. Acetaminophen (500-650 mg PO q 4-6 hours) should be continued as needed, and should not exceed 3000 mg/day.

### Maintenance Therapy: 10 MIU/m² SC TIW for 48 weeks.

After induction therapy, subjects will continue on maintenance therapy and receive INTRON® A, 10 million international units/m²/day, SC three times weekly for 48 weeks.

### Group B: PEG Intron: PEG₁₂₀₀₀-interferon alfa-2b, 6.0 µg/kg, SC once weekly for 2 years.

Subjects randomized to treatment Group B will receive PEG₁₂₀₀₀-interferon alfa-2b, 6.0 µg/kg, SC once weekly for 2 years. Acetaminophen (500-1000 mg) may be given in the clinic 30 minutes prior to receiving the first dose of PEG Intron. Subjects should be observed for 2 hours after the first dose. Acetaminophen (500-650 mg PO q 4-6 hours) should be continued as needed, and should not exceed 3000 mg/day.

### Duration of Study and Visit Schedule

Treatment with either PEG₁₂₀₀₀-interferon alfa 2b (about 104 weeks) or INTRON® A (52 weeks) will continue as scheduled unless there is evidenced of disease recurrence, unacceptable toxicity, or the subject requests that therapy be discontinued. Tolerability of the respective study treatment and quality of life will be assessed from clinical observation, routine lab oratory testing, and quality of life assessments over the course of therapy. Following completion of therapy, subjects will continue to be followed for evidenced of disease recurrence and will complete quality of life assessments. If the melanoma recurs, further treatment will be at the discretion of the physician. All subjects will be followed for survival, regardless of when they discontinue therapy. Analyses of relapse-free and overall survival, regardless of when they discontinue therapy. Analyses of relapse-free and overall survival will be event driven.

The duration of this study is based upon achieving a therapeutic response, and will be determined for each subject individually.

The study population will include male and female patients with cutaneous melanoma and will be included if they meet the following inclusion and exclusion criteria:

### Subject Inclusion Criteria

A subject is eligible to participate in this study if he or she:
a) Subjects must have histologically documented primary cutaneous melanoma meeting one of the following staging criteria:
   - Primary melanoma of any stage in the presence of N1 regional lymph node metastases detected at elective lymph node dissection or sentinel node biopsy, with clinically inapparent regional lymph node metastasis (any pTN₁M₀).
   - Clinically apparent N1 or N2a regional lymph node involvement synchronous with primary melanoma of T₁₋₄ (any pTrN₁₋₂ₐM₀).
   - Regional lymph node recurrence at any interval after appropriate surgery for primary melanoma of any depth (any pTrN₁₋₂ₐM₀).
b) Subjects must have had all known disease completely resected with adequate surgical margins within 56 days prior to randomization into the study.
c) Subjects must have an ECOG performance status of 0 or 1 as defined by Minna, J D, et al. "Cancer of the Lung" in DeVita V, et al. eds., Cancer: Principles and Practiced of Oncology, Lippincott, Philadelphia, PA 1989 at page 536.
d) Subjects must be between 18-70 years old.
e) Subjects must have adequate hepatic, renal and bone marrow function as defined by the following parameters obtained within 14 days prior to initiation of study treatment.
   1) Hematology:
      - White Blood count (WBC) ≥3,000 cells/µL.
      - Hemoglobin concentration ≥9g/dL.
   2) Renal and hepatic function:
      - Serum creatinine ≤2.0 mg/dL or calculated creatinine clearance of ≥50 mL/minute.
      - Serum bilirubin <2 times the upper limit of normal (ULN), unless due to infiltration by disease.
      - AST/ALT (SGOT/SGPT) <2 times ULN.
f) has submitted a written voluntary informed consent before study entry, is willing to participate in this study and will complete all follow up assessments.

### Subject Exclusion Criteria

A subject is not eligible to participate in this study if he or she:
a) Subjects who have received any prior chemotherapy, immunotherapy hormonal or radiation therapy for melanoma.
b) Subjects who have evidence of distant or non-regional lymph node metastases, in-transit metastases, or positive lymph nodes with an unknown primary.
c) Subjects whose disease cannot be completely surgically resected because of gross extracapsular extension.
d) Subjects who have previously received interferon-α for any reason. (Such patients however, are still considered treatable in accordance with the method of this invention but are only excluded from this registration study.)
e) Subjects who have severe cardiovascular disease, i.e., arrhythmias requiring chronic treatment, congestive heart failure (NYHA Class III or IV) or symptomatic ischemic heart disease as defined by Bruce RA: Evaluation of Functional Capacity and Exercise Tolerance of Cardiac Subjects" in Mod. Concepts Cardiovasc Dis 1956; 25-321.
f) Subjects who have a history of neuropsychiatric disorder requiring hospitalization.
g) Subjects with thyroid dysfunction not responsive to therapy.
h) Subjects with uncontrolled diabetes mellitus.
I) Subjects with a history of prior malignancy within the past 5 years other than surgically cured non-melanoma skin cancer or cervical carcinoma in situ.
j) Subjects who have a history of seropositivity for HIV.
k) Subjects who are pregnant, lactating, or of reproductive potential and not practicing an effective means of contraception.
l) Subjects with active and/or uncontrolled infection, including active hepatitis.
m) Subjects with a medical condition requiring chronic systemic corticosteroids.
n) Subjects who are known to be actively abusing alcohol or drugs.
o) Subjects who have received any experimental therapy within 30 days prior to randomization in this study.
p) Subjects who have not recovered from the effects of recent surgery.

### Subject Discontinuation Criteria

It is the right and duty of the clinical investigator to interrupt the treatment of any subject whose health or well being may be threatened by continuation in this study.

Subjects may be discontinued prior to completion of this study for any of the following reasons:
a) Develops documented progression or recurrence of disease, as defined herein above.
b) Has a clinically significant adverse event as determined by the Principal Investigator.
c) Requests to be withdrawn from the study.
d) Is unable to complete the study evaluations/visits because of unforeseen circumstances.
e) Develops other conditions for which, in the investigator's opinion warrants withdrawal from the study
f) Develops severe depression or any other psychiatric disorder requiring hospitalization.
g) Experiences a serious allergic response to the study drug manifested by angioedema, bronchoconstriction or anaphylaxis.
h) Receives treatment with a prohibited medication as indicated herein above.
l) Experiences recurrent toxicities despite dose modifications as described herein below.

All subjects will be followed for survival, regardless of when they go off study. Subjects who discontinue for reasons other than recurrence of disease should also be followed for recurrence and survival.

### Analysis of Primary and Secondary Endpoints

The primary endpoint will be progression-free survival (PFS) time, defined to be the time from randomization to progression or death. PFS will be assessed by clinical observation, with recurrence documented by appropriate radiographic and histologic methods, and confirmed by Independent Central Review.

The secondary endpoints will be overall survival, safety, quality of life, and population pharmacokinetics (PK). Safety and tolerability will be assessed from clinical observation and routine laboratory testing over the course of therapy. Health-Related Quality of Life (HQL) will be assessed from an HQL questionnaire.
Population pharmacokinetics will be assessed from periodic serum sampling in the PEG Intron group.

Subjects enrolled in Group A who are not able to tolerate the IV induction dose regimen despite dose modification, should stop the IV regimen but should not be discontinued from the study. After resolution of toxicity, they may enter the INTRON® A maintenance phase with the full maintenance dose.

## Claims

1. The use of pegylated interferon alpha-2b for the manufacture of a medicament for treatment of a patient having melanoma, wherein said medicament is to be administered once a week in a therapeutically effective dose of pegylated interferon alpha-2b for a time period sufficient to increase progression-free survival time, wherein the therapeutically effective dose is in the range of about 3.0 µg/kg to about 9.0 µg/kg.

2. The use according to claim 1, wherein the therapeutically effective dose is in the range of about 4.5 µg/kg to about 6.5 µg/kg.

3. The use according to claim 2, wherein the therapeutically effective dose is in the range of about 5.5 µg/kg to about 6.5 µg/kg.

4. The use according to claims 3, wherein the therapeutically effective dose is about 6.0 µg/kg.

5. The use according to any one of claims 1-4, wherein the time period is a minimum of 100 to 104 weeks.

6. The use according to claim 5, wherein the time period is up to five years.

7. The use of pegylated interferon alpha-2b for the manufacture of a medicament for treatment of a patient having melanoma, wherein said medicament is to be administered once a week at 6.0 µg/kg of pegylated interferon alpha-2b for an initial time period of 8 weeks and 3.0 µg/kg or less once a week for 252 weeks.

8. The use according to any one of claims 1-7, wherein the pegylated interferon alpha-2b comprises polyethylene glycol having an average molecular weight of 12,000.

9. The use according to any one of claims 1-8, wherein the polyethylene glycol moiety is conjugated to the interferon alpha-2b via a urethane linkage.

10. The use according to any one of claims 1-9, wherein the pegylated interferon alpha-2b is formulated as a lyophilized powder for injection.

11. The use according to any one of claims 1-10, wherein the patient is a treatment-naive patient.

12. The use according to any one of claims 1-10, wherein the patient is a treatment-experienced patient, who is intolerant to interferon alpha or resistant to interferon alpha.

13. The use according to any one of claims 1-12, wherein said melanoma is Stage IIB or Stage III cutaneous melanoma.

## Patentansprüche

1. Verwendung von pegyliertem Interferon alpha-2b zur Herstellung eines Arzneimittels zur Behandlung eines Patienten mit Melanom, wobei das Medikament in einer therapeutisch wirksamen Dosis an pegyliertem Interferon alpha-2b einmal pro Woche für eine Dauer zu verabreichen ist, welche die Progressions-freie Überlebenszeit steigert, wobei das die therapeutisch wirksame Dosis im Bereich von etwa 3,0 µg/kg bis etwa 9,0 µg/kg liegt.

2. Verwendung gemäß Anspruch 1, bei der die therapeutisch wirksame Dosis im Bereich von etwa 4,5 µg/kg bis etwa 6,5 µg/kg liegt.

3. Verwendung gemäß Anspruch 2, bei der die therapeutisch wirksame Dosis im Bereich von etwa 5,5 µg/kg bis etwa 6,5 µg/kg liegt.

4. Verwendung gemäß Anspruch 3, bei der die therapeutisch wirksame Dosis etwa 6,0 µg/kg ist.

5. Verwendung gemäß einem der Ansprüche 1 bis 4, bei der die Dauer mindestens 100 bis 104 Wochen beträgt.

6. Verwendung gemäß Anspruch 5, bei der die Dauer bis zu 5 Jahre beträgt.

7. Verwendung von pegyliertem Interferon alpha-2b zur Herstellung eines Arzneimittels zur Behandlung eines Patienten mit Melanom, wobei das Medikament einmal pro Woche mit 6,0 µg/kg an pegyliertem Interferon alpha-2b für eine Anfangszeitdauer von 8 Wochen und einmal pro Woche mit 3,0 µg/kg oder weniger für 252 Wochen zu verabreichen ist.

8. Verwendung gemäß einem der Ansprüche 1 bis 7, bei der das pegylierte Interferon alpha-2b Polyethylenglykol mit einem durchschnittlichen Molekulargewicht von 12.000 umfasst.

9. Verwendung gemäß einem der Ansprüche 1 bis 8, bei der die Polyethylenglykol-Gruppe an das Interferon alpha-2b über eine Urethan-Bindung konjugiert ist.

10. Verwendung gemäß einem der Ansprüche 1 bis 9, bei der das pegylierte Interferon alpha-2b als lyophilisiertes Pulver für die Injektion formuliert ist.

11. Verwendung gemäß einem der Ansprüche 1 bis 10, bei der der Patient ein unbehandelter Patient ist.

12. Verwendung gemäß einem der Ansprüche 1 bis 10, bei der der Patient ein zuvor behandelter Patient ist, der gegenüber Interferon alpha intolerant oder resistent ist.

13. Verwendung gemäß einem der Ansprüche 1 bis 12, bei der das Melanom kutanes Melanom der Stufe IIB oder der Stufe III ist.

## Revendications

1. Utilisation d'un interféron alpha-2b pégylé pour la fabrication d'un médicament pour le traitement d'un patient ayant un mélanome, dans laquelle ledit médicament est à administrer à une dose thérapeutiquement efficace d'interféron alpha-2b pégylé une fois par semaine pendant une période suffisante pour augmenter le temps de survie sans aggravation, dans lequel la dose efficace thérapeutique est d'environ 3µg/kg à environ 9µg/kg.

2. Utilisation selon la revendication 1, dans laquelle la dose efficace thérapeutique est comprise entre environ 4,5µg/kg à environ 6,5µg/kg.

3. Utilisation selon la revendication 2, dans laquelle la dose thérapeutiquement efficace est comprise entre environ 5,5µg/kg et environ 6,5µg/kg.

4. Utilisation selon la revendication 3, dans laquelle la dose thérapeutiquement efficace est d'environ 6µg/kg.

5. Utilisation selon l'une quelconque des revendications 1 à 4, dans laquelle la période de temps est au minimum de 100 à 104 semaines.

6. Utilisation selon la revendication 5, dans laquelle la période de temps va jusqu'à cinq ans.

7. Utilisation d'un interféron alpha-2b pégylé pour la fabrication d'un médicament pour le traitement d'un patient ayant un mélanome, dans laquelle ledit médicament est à administrer une fois par semaine à la dose de 6µg/kg d'interféron alpha-2b pégylé pendant une période de temps initiale de 8 semaines et de 3µg/kg ou moins une fois par semaine pendant 252 semaines.

8. Utilisation selon l'une quelconque des revendications 1 à 7, dans laquelle l'interféron alpha-2b pégylé comprend du polyéthylène glycol ayant un poids moléculaire moyen de 12 000.

9. Utilisation selon l'une quelconque des revendications 1 à 8, dans laquelle la partie polyéthylène glycol est conjuguée à l'interféron alpha-2b via une liaison uréthane.

10. Utilisation selon l'une quelconque des revendications 1 à 9, dans laquelle l'interféron alpha-2b pégylé est formulé comme une poudre lyophilisée pour injection.

11. Utilisation selon l'une quelconque des revendications 1 à 10, dans laquelle le patient est un patient naïf pour le traitement.

12. Utilisation selon l'une quelconque des revendications 1 à 10, dans laquelle le patient est un patient ayant déjà été traité, qui est intolérant à l'interféron alpha ou résistant à l'interféron alpha.

13. Utilisation selon l'une quelconque des revendications 1 à 12, dans laquelle ledit mélanome est un mélanome cutané de stade IIB ou de stade III.
